# EUROPEAN PATENT SPECIFICATION

(11) **EP 3 512 877 B1**
(45) Date of publication and mention of the grant of the patent: **26.03.2025**
(21) Application number: 17772626.2
(22) Date of filing: 13.09.2017
(51) Int. Cl.: C07K 14/78, C12Q 1/37, G01N 33/574

(54) **NIDOGEN-1 FRAGMENTS ASSAY**
NIDOGEN-1-FRAGMENTE-TEST
DOSAGE DE FRAGMENTS DE NIDOGÈNE -1

(30) Priority: 15.09.2016 GB 201615746
(43) Date of publication of application: 24.07.2019
(73) Proprietor: Nordic Bioscience A/S, 2730 Herlev (DK)
(72) Inventor: WILLUMSEN, Nicholas, 2870 Dyssegard (DK); BAGER, Cecilie, Liv, 1799 Kobenhavn V (DK); BAY JENSEN, Anne-Cecilie, 2300 Copenhagen S (DK); QUILEZ-ALVAREZ, Antonio, 28005 Madrid (ES); KARSDAL, Morten, DK-2100 København Ø (DK); ORSNES-LEEMING, Diana, 2730 Herlev (DK); MANON-JENSEN, Tina, DK-2605 Brondby (DK)
(74) Representative: Beck Greener LLP
(86) International application number: PCT/EP2017/072973
(87) International publication number: WO 2018/050671

(56) References cited:
- WO-A1-2011/119484
- WO-A1-2016/172722
- WO-A2-2013/040142
- US-A1- 2012 045 781
- LIN LI ET AL: "Nidogen-1: a candidate biomarker for ovarian serous cancer", JAPANESE JOURNAL OF CLINICAL ONCOLOGY., vol. 45, no. 2, 6 November 2014 (2014-11-06), JP, pages 176 - 182, XP055419654, ISSN: 0368-2811, DOI: 10.1093/jjco/hyu187
- JULIETTE SAGE ET AL: "Cleavage of Nidogen-1 by Cathepsin S Impairs Its Binding to Basement Membrane Partners", PLOS ONE, vol. 7, no. 8, 28 August 2012 (2012-08-28), pages e43494, XP055419239, DOI: 10.1371/journal.pone.0043494
- RAGHU KALLURI: "Angiogenesis: Basement membranes: structure, assembly and role in tumour angiogenesis", NATURE REVIEWS. CANCER, vol. 3, no. 6, 1 June 2003 (2003-06-01), GB, pages 422 - 433, XP055419604, ISSN: 1474-175X, DOI: 10.1038/nrc1094
- LISA SEVENICH ET AL: "Analysis of tumour- and stroma-supplied proteolytic networks reveals a brain-metastasis-promoting role for cathepsin S", NATURE CELL BIOLOGY, vol. 16, no. 9, 3 August 2014 (2014-08-03), GB, pages 876 - 888, XP055419213, ISSN: 1465-7392, DOI: 10.1038/ncb3011
- REIK LÖSER ET AL: "Cysteine cathepsins: their role in tumor progression and recent trends in the development of imaging probes", FRONTIERS IN CHEMISTRY, vol. 3, 23 June 2015 (2015-06-23), XP055419297, DOI: 10.3389/fchem.2015.00037
- GONG-JUN TAN ET AL: "Cathepsins mediate tumor metastasis", 26 November 2013 (2013-11-26), pages 91 - 101, XP055419599, Retrieved from the Internet <URL:https://www.ncbi.nlm.nih.gov/pmc/articles/PMC3856311/pdf/WJBC-4-91.pdf> [retrieved on 20171026], DOI: 10.4331/wjbc.v4.i4.91
- NICHOLAS WILLUMSEN ET AL: "Nidogen-1 Degraded by Cathepsin S can be Quantified in Serum and is Associated with Non-Small Cell Lung Cancer", NEOPLASIA, vol. 19, no. 4, 1 April 2017 (2017-04-01), US, pages 271 - 278, XP055419235, ISSN: 1476-5586, DOI: 10.1016/j.neo.2017.01.008
- ULRIKE MAYER ET AL: "Sites of nidogen cleavage by proteases involved in tissue homeostasis and remodelling", EUROPEAN JOURNAL OF BIOCHEMISTRY, vol. 217, no. 3, 1 November 1993 (1993-11-01), GB, pages 877 - 884, XP055419595, ISSN: 0014-2956, DOI: 10.1111/j.1432-1033.1993.tb18316.x

## Description

### Field of the invention

The present application relates to an assay for measuring fragments of Nidogen-1 having an N- or C-terminal neo-epitope formed by cleavage of Nidogen-1 by Cathepsin-S, and the use of said assay for the diagnosis of cancer and for evaluating anti-cancer drugs.

### Background

A major component of the tumor microenvironment is the extracellular matrix (ECM). Disruption of the healthy tissue homeostasis and altered turnover of the ECM are hallmarks of cancer that may lead to progression of the disease (1). The basement membrane (BM) is a specialized layer of ECM that underlies and surrounds epithelial cells, endothelial cells and mesenchymal cells and is essential for embryonic development as well as for maintaining tissue architecture and cellular polarization (2). The BM also serves as a barrier for cell invasion and breaching of the BM, and loss of BM integrity has been associated with an invasive phenotype in cancer (3).

Nidogens are highly conserved proteins that are found in almost all BMs (4). Two different nidogens exist: nidogen-1 (entactin) and nidogen-2. Nidogen-1 is the most widely expressed and comprises three globular domains (G1, G2 and G3) connected by two rod-shaped domains. Nidogen-1 serves as the linker between other BM proteins, namely collagen type IV, perlecan and laminin (5). Nidogen-1 also plays a key role in the BM assembly and stabilization (6) as is evident by the lung and heart abnormalities and perinatal death observed in nidogen-deficient mice as a direct result of BM changes (7).

It has also been shown that nidogen-1 can regulate specific gene-expression in mammary epithelial cells (8). When the expression of β-casein was investigated as a function of nidogen quality/composition, a fragment of nidogen containing the laminin-1 binding domain, but lacking the type IV collagen binding domain was able to reduce the expression of β-casein. In contrast, a fragment of nidogen containing the type IV collagen binding domain, but lacking the laminin-1 binding domain was not. This indicates that in addition to maintaining BM integrity, nidogen has a physiological role in BM-induced gene expression and this is influenced by the quality/composition of nidogen. Additionally, Kalluri (22) discloses that intact Nidogen-1 in the plasma can be used as a marker for ovarian serous cancer.

Lin Li et al. (24) discloses a general diagnostic link between intact Nidogen-1 in the plasma and ovarian serous carcinoma. There is no discussion of cleavage products of Nidogen-1, in particular Cathepsin-S (Cats) cleavage of Nidogen-1. Lin Li et al. fails to disclose a method of evaluating the efficacy of an anti-cancer drug; there is only a brief speculative comment in the abstract regarding potential cancer therapies. Sage et al. (9) showed that Nidogen-1 is a substrate for cathespin-S (CatS), and that CatS is found in both normal and tumor tissues. However, the physiological relevance of nidogen cleavage by CatS in normal and tumor tissues remained unknown. US 2012/045781 discloses methods of diagnosis or of quantitation of pathological conditions which comprise conducting an immunoassay to measure neo-epitope containing protein fragments naturally present in a biofluid sample. The disclosed neo-epitopes are fragments of collagen type I, collagen type III, collagen type IV, collagen type V, collagen type VI, elastin, biglycan, decorin, lumican, versican, C-reactive protein, ApoE and laminins. Further methods of peptide identification are disclosed in WO 2011/119484 and WO 2013/040142.

It is thought that cathepsins may play an important role in cancer (10 and 23). As noted in Sage et al. CatS is produced in normal tissues and tumour tissues, and it has also been found that CatS is produced by tumor-associated macrophages (TAMs) (11). CatS has been linked to growth, angiogenesis, migration, invasion and mestatasis in different cancer types (11-14). However, Kos et al (21) showed that the role of CatS in cancer progression is strongly associated with an immune response rather than with the remodelling of the extracellular matrix.

### Summary of the invention

The applicant has found that nidogen-1 degraded by CatS has biomarker potential in cancer. Without being bound by theory, it is hypothesized that this may be a reflection of a loss of BM integrity, a phenomenon which is known to be associated with pro-cancer events and an invasive phenotype. Accordingly, an aim of the present invention was to enable non-invasive assessment of cancer using biomarkers produced by CatS degradation of nidogen-1.

In a first aspect, the present invention relates to a method of diagnosis or of quantitation of cancer comprising conducting an immunoassay on a biofluid sample obtained from a patient to measure fragments of Nidogen-1 having an N- or C-terminal neo-epitope formed by cleavage of Nidogen-1 by Cathepsin-S, said fragments being naturally present in said sample, and associating an elevation of said measure in said patient above a normal level with the presence or extent of cancer, wherein said immunoassay is conducted by a method comprising:
contacting the fragments of Nidogen-1 having said N- or C-terminal neo epitope that are naturally present in said sample with an immunological binding partner specifically reactive with the N- or C-terminal neo-epitope but not reactive with intact Nidogen-1, and measuring the extent of binding of the N- or C-terminal neo-epitope to said immunological binding partner to measure therein fragments comprising said neo-epitope.

The cancer may be breast cancer or non-small cell lung cancer (NSCLC) .

Preferably, the immunological binding partner is specifically reactive with a C-terminal neo-epitope selected from the group consisting of:
...CQHERE (SEQ ID NO: 1)
...YSLLPL (SEQ ID NO: 2)
...IIRQDL (SEQ ID NO: 3)
or is specifically reactive with an N-terminal neo-epitope selected from the group consisting of:
APVGGI... (SEQ ID NO: 4)
HILGAA... (SEQ ID NO: 5)
GSPEGI... (SEQ ID NO: 6)

Preferably, an immunological binding partner specifically reactive with a C-terminal neo-epitope does not react with a truncated and/or elongated C-terminal sequence at the C-terminus. Similarly, an immunological binding partner specifically reactive with an N-terminal neo-epitope preferably does not react with a truncated and/or elongated N-terminal sequence at the N-terminus. "Elongated" as used herein means the sequence is elongated by one or more amino acids. "Truncated" as used herein means the sequence is truncated by one or more amino acids. In a preferred embodiment of the invention, the immunological binding partner is specifically reactive with the C-terminal neo-epitope VEKTRCQHERE-COOH (SEQ ID NO: 7). Preferably, the immunological binding partner does not react with a truncated C-terminal sequence VEKTRCQHER-COOH (SEQ ID NO: 8) and/or wherein the immunological binding partner does not react with an elongated C-terminal sequence VEKTRCQHEREH-COOH (SEQ ID NO: 9).

The immunological binding partner may be a polyclonal antibody or a monoclonal antibody.

The diagnostic method described above may be used to differentiate between a patient with NSCLC and a patient with another cancer type. In this regard the method preferably comprises performing the method as described above and associating an elevation of said measure in said patient of at least 30% above a normal level with the presence of NSCLC.

In another aspect, the present invention relates to a method for evaluating the efficacy of an anti-cancer drug, wherein said method comprises
conducting an immunoassay to quantify the amount of fragments of Nidogen-1 having an N- or C-terminal neo-epitope formed by cleavage of Nidogen-1 by Cathepsin-S in at least two biological samples, said biological samples having been obtained from a subject at a first time point and at at least one subsequent time point during a period of administration of the anti-cancer drug to said subject, and wherein a reduction in the quantity of said fragments of Nidogen-1 having an N- or C-terminal neo-epitope formed by cleavage of Nidogen-1 by Cathepsin-S from said first time point to said at least one subsequent time point during the period of administration of the anti-cancer drug is indicative of an efficacious anti-cancer drug,
wherein said immunoassay is conducted by a method comprising contacting the fragments of Nidogen-1 having said N- or C-terminal neo epitope that are naturally present in said samples with an immunological binding partner specifically reactive with the N- or C-terminal neo-epitope but not reactive with intact Nidogen-1, and measuring the extent of binding of the N- or C-terminal neo-epitope to said immunological binding partner to measure therein fragments comprising said neo-epitope.

Preferably, the immunological binding partner is as described *supra.*

In a further aspect the present invention relates to a diagnostic kit for use in the methods described *supra,* the kit comprising an immunological binding partner specifically reactive with an N- or C-terminal neo-epitope formed by cleavage of Nidogen-1 by Cathepsin-S but not reactive with intact Nidogen-1, and at least one of the following:
- a streptavidin coated 96 well plate
- a biotinylated peptide corresponding to the amino acid sequence of the N- or C-terminal neo-epitope, with an optional linker located between the biotin residue and the peptide
- a biotinylated secondary antibody for use in a sandwich immunoassay
- a calibrator peptide corresponding to the amino acid sequence of the N- or C-terminal neo-epitope
- an antibody HRP labeling kit
- an antibody radiolabeling kit
- an assay visualization kit

Preferably, the assay kit comprises a biotinylated peptide Biotin-L-VEKTRCQHERE-COOH (SEQ ID NO: 10), wherein L is an optional linker, and a calibrator peptide comprising the C-terminal sequence VEKTRCQHERE-COOH (SEQ ID NO: 11).

### Definitions

The term "antibody" is used according to the invention in the broadest sense and specifically covers intact monoclonal antibodies, polyclonal antibodies, and antibody fragments, so long as they exhibit the desired biological activity.

"Antibody fragments" used in the invention comprise a portion of an intact antibody, preferably comprising the antigen-binding or variable region thereof. Examples of antibody fragments include Fab, Fab', F(ab')2, Fv and Fc fragments.

"Fragments of Nidogen-1" used in the invention means a peptide fragment produced by CatS cleavage of Nidogen-1. "N- or C-terminal neo-epitope" used in the invention means an N- or C-terminal epitope formed at a CatS cleavage site of Nidogen-1. For example, the following sequence of Nidogen-1

...CQHERE↓HILGAA...

would produce the N-terminal neo-epitope H₂N-HILGAA... (SEQ ID NO: 5) and the C-terminal neo-epitope ...CQHERE-COOH (SEQ ID NO: 1) when cleaved by CatS at the site between the E⁶⁹³-H⁶⁹⁴ peptide bond, as denoted by the symbol "↓".

The term "a normal level" is used herein to mean a level/amount consistent with healthy individuals in a population. This generally corresponds to the median value in control samples ± standard deviation.

"NIC" as used herein refers to fragments of Nidogen-1 comprising the C-terminal neo-epitope VEKTRCQHERE-COOH (SEQ ID NO: 7).

### Figures

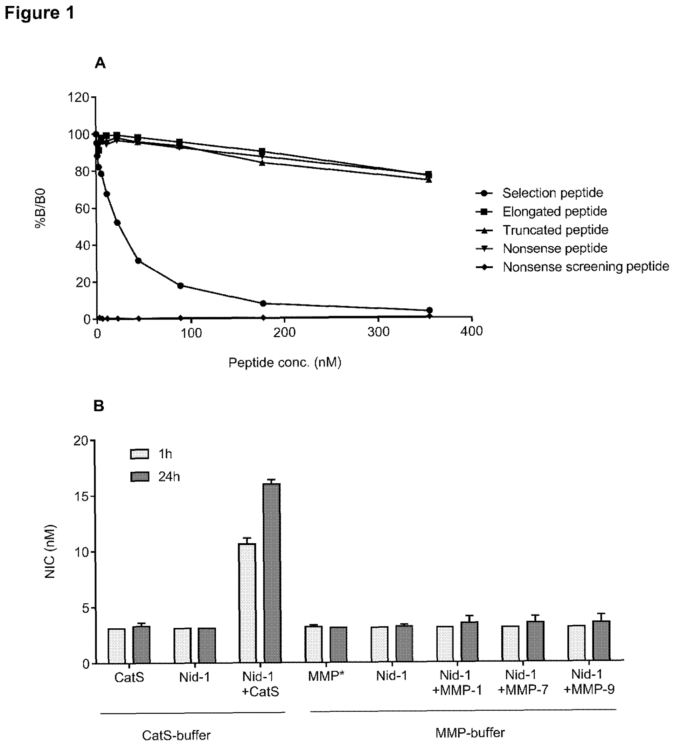

Figure 1: Specificity of the NIC assay. A) the percentage of inhibition at given concentrations in the competitive NIC- 2 ELISA tested with the selection peptide (VEKTRCQHERE; SEQ ID NO: 11), an elongated selection peptide (VEKTRCQHEREH; SEQ ID NO: 16), a truncated selection peptide (EVEKTRCQHER; SEQ ID NO: 15), a non-sense selection peptide (APVGGIIGWM; SEQ ID NO: 12) and a non-sense screening peptide (Biotin-APVGGIIGWM; SEQ ID NO: 13). %B/B0: B equals the OD at x nM peptide and B0 equals the OD at 0 nM peptide. B) NIC levels measured after cleavage of human recombinant nidogen-1 (Nid-1) with Cathepsin S (CatS), and matrix metalloprotease (MMP) -1, -7 and -9. Values below the lower level of detection (LLOD) were assigned the LLOD. *representative of both MMP-1, -7 and -9.

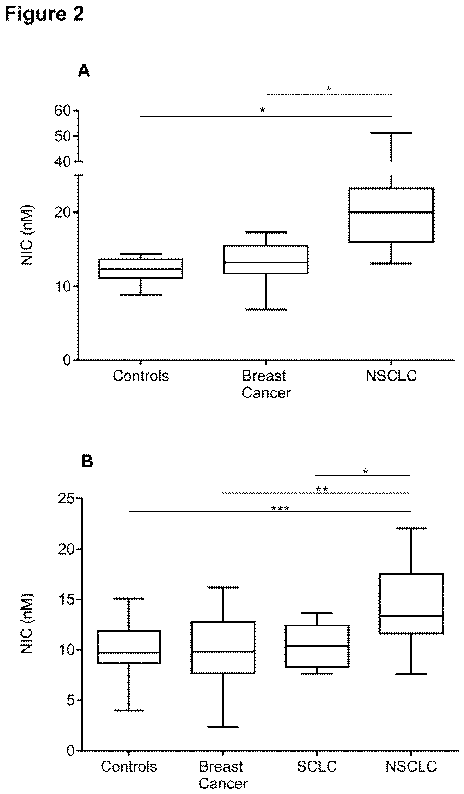

Figure 2: Evaluation of NIC in serum from two study cohorts. A) Proteogenex samples; B) Asterand samples). Box and whiskers show data from minimum to maximum. Data were compared by one-way ANOVA adjusted for multiple comparisons.*p<0.05, **p<0.01, ***p<0.001. NSCLC: non-small cell lung cancer; SCLC: small cell lung cancer.

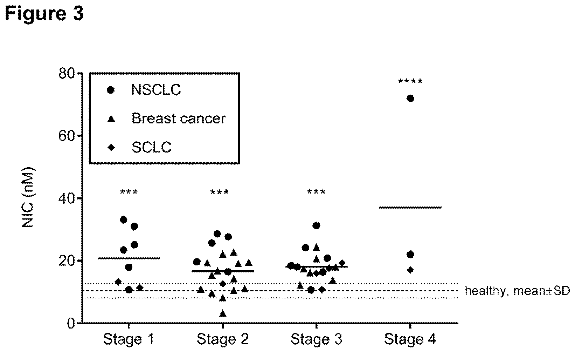

Figure 3: Evaluation of NIC in serum according to tumor stage. Samples were pooled according to tumor stage. Data are presented as scatter plots with each cancer type identifiable. Data were compared to healthy controls by one-way ANOVA adjusted for multiple comparisons. ***p<0.001, ****p<0.0001. NSCLC: non-small cell lung cancer; SCLC: small cell lung cancer.

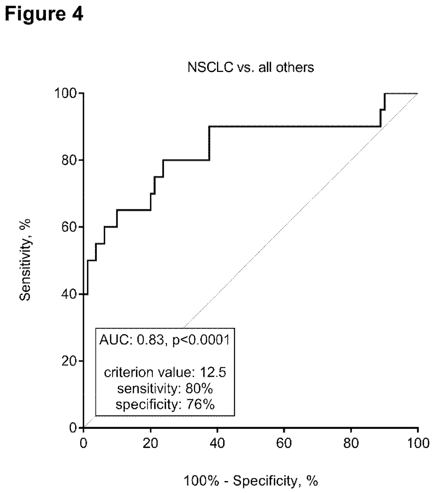

Figure 4: Receiver operating characteristics (ROC) analysis evaluating the ability of NIC to separate NSCLC from all other samples included in this study combined (breast cancer, SCLC and healthy controls). AUC: area under the ROC curve; NSCLC: non-small cell lung cancer; SCLC: small cell lung cancer

### DETAILED EXAMPLE OF A PREFERRED EMBODIMENT OF THE INVENTION

### MATERIALS AND METHODS

### Selection of peptides

The following three main CatS cleavage-sites (↓) on nidogen-1 were previously identified by N-terminal sequencing (9):
1) NH₃-...LLPL⁴⁵⁹↓APVG..., located within the central part of the G2 domain;
2) NH₃-...HERE⁶⁹³↓HILG... located in the thyroglobulin-like (Tg) domain adjacent to the G3 domain; and
3) NH₃-...RQDL⁸⁵⁹↓GSPE..., located at the N-terminal part of the G3 domain.

The proteolytic cleavage corresponding to the C-terminal neo-epitope ...HERE⁶⁹³ originating from outside the globular domains in the direction towards the N-terminus was selected for raising antibodies and development of immunoassays.

To generate an antibody specific for the selected CatS cleavage site on nidogen-1 a sequence of 11 amino acids adjacent to the cleavage site was chosen as the target: VEKTRCQHERE↓ (SEQ ID NO: 7). This amino acid sequence was used to design the selection peptide. The sequence was blasted for homology to other human proteins using NPS@: Network Protein Sequence Analysis with the UniprotKB/Swiss-prot database (15) and found to be unique to human nidogen-1.

A biotinylated screening peptide (Biotin-VEKTRCQHERE; SEQ ID NO: 10) was included for coating streptavidin-coated plates. To test for specificity of the antibody a truncated selection peptide (EVEKTRCQHER; SEQ ID NO: 15) missing the one amino acid closest to the cleavage site, and an elongated selection peptide (VEKTRCQHEREH; SEQ ID NO: 16), with an additional amino acid added C-terminally to the cleavage site, as well as a non-sense selection peptide (APVGGIIGWM; SEQ ID NO: 12) and a non-sense biotinylated screening peptide (Biotin-APVGGIIGWM; SEQ ID NO: 13) were included in the assay development. The latter two correspond to the cleavage site located within the central part of the G2 domain (↓APVG...). The immunogenic peptide (KLH-VEKTRCQHERE; SEQ ID NO: 14) was generated by covalently cross-linking the selection peptide to Keyhole Limpet Hemocyanin (KLH) carrier protein using Succinimidyl 4-(N-maleimidomethyl)cyclohexane-1-carboxylate, SMCC (Thermo Scientific, Waltham, MA, USA, cat. #22336). The synthetic peptides used for NIC are captured in Table 1.

| Table 1. Amino acid sequence of the synthetic peptides used for antibody production and assay validation | |
|---|---|
| Selection peptide | VEKTRCQHERE (SEQ ID NO: 11) |
| Immunogenic peptide | *KLH-VEKTRCQHERE (SEQ ID NO: 14) |
| Screening peptide | Biotin-VEKTRCQHERE (SEQ ID NO: 10) |
| Truncated selection peptide | EVEKTRCQHER (SEQ ID NO: 15) |
| Elongated selection peptide | VEKTRCQHEREH (SEQ ID NO: 16) |
| Nonsense selection peptide | APVGGIIGWM (SEQ ID NO: 12) |
| Nonsense screening peptide | Biotin-APVGGIIGWM (SEQ ID NO: 13) |
| *Keyhole Limpet Hemocyanin | |

All synthetic peptides were purchased from the American Peptide Company, Vista, CA, USA.

### Antibody development

Six week old BALB/c mice (n=6) were immunized subcutaneously with 200 µL emulsified antigen and 50 µg of the immunogenic peptide (KLH-VEKTRCQHERE; SEQ ID NO: 14) using Freund's incomplete adjuvant (Sigma-Aldrich, St. Louis, MO, USA). Immunizations were repeated every second week and blood samples collected from the second immunization until stable serum antibody titer levels were reached. The mice with the highest antibody titer rested for a month and were then boosted intravenously with 50 µg immunogenic peptide in 100 µL 0.9% sodium chloride solution. Three days later the spleen was isolated for cell fusion. The fusion procedure was performed as described elsewhere (16). Briefly, mouse spleen cells were fused with SP2/0 myeloma cells to produce hybridoma cells. The hybridoma cells were cloned in culture dishes and limited-dilution was used to secure monoclonal growth. Native reactivity and peptide binding of the monoclonal antibodies were evaluated by displacement using human serum samples and the selection-peptide (VEKTRCQHERE; SEQ ID NO: 11) in a preliminary ELISA using 10 ng/mL screening peptide on a streptavidin-coated microtiter plate (Roche, Basel, Switzerland, cat. #11940279) and the supernatant from the growing monoclonal hybridoma cells (containing the antibodies). The clones with best reactivity were purified using protein-G-columns according to the manufacturer's instructions (GE Healthcare Life Sciences, Little Chalfont, UK, cat. #17-0404-01). Two hybridoma clones qualified to a screening for their ability to react competitively only with the selection peptide (VEKTRCQHERE; SEQ ID NO: 11) and not with the truncated (EVEKTRCQHER; SEQ ID NO: 15), elongated (VEKTRCQHEREH; SEQ ID NO: 16) and non-sense (APVGGIIGWM; SEQ ID NO: 12) selection peptides. One hybridoma clone (NB613-12) was selected for further assay development. Optimal incubation-buffer, incubation-time and incubation-temperature, as well the optimal concentrations of antibody and screening peptide, were determined from checkerboard analysis.

### Proteolysis of nidogen-1 in vitro

Human recombinant (hr-) nidogen-1 (R&D Systems, Minneapolis, MN, USA, cat. #2570-ND) was dissolved in CatS-buffer (100 mM NaH₂PO₄(H₂O)₂, 2 mM DTT, 0.01% Brij-35, pH 7.4) or MMP-buffer (50 mM Tris, 150 mM NaCl, 10 mM CaCl₂, 10 uM ZnCl, pH 7.5) to a final concentration of 125 µg/ml. The nidogen-1 solutions incubated at 37°C for 1h and 24h with, or without, the addition of hr-cathepsin S (Calbiochem, Whitehouse Station, NJ, USA, cat. #219343), hr-MMP-1 (R&D Systems, cat. #901-MP), hr-MMP-7 (R&D Systems, cat. #907-MP) or hr-MMP-9 (R&D Systems, cat. #911-MP) in final concentrations of 1.25 µg/ml, resulting in an enzyme-to-protein ratio of 1:100. The chosen MMPs had previously been shown to degrade nidogen-1. (17,18) Enzymatic activity tests were performed in parallel with the actual cleavage of nidogen-1. The reaction was stopped by adding E-64 or EDTA (final concentration of 1 µM) to CatS- and MMP-solutions, respectively. CatS- and MMP-buffer with relevant proteases were included as controls. The experiment was repeated twice. Samples were stored at -80°C until analysis. The cleavage of nidogen-1 was confirmed by silverstaining according to the manufacturer's instructions (SilverXpress^{®}, Invitrogen, cat. #LC6100) (data not shown).

### NIC assay protocol

The competitive ELISA procedure was as follows: a 96-well streptavidin-coated microtiter plate, was coated with 2.5 ng/ml screening-peptide (Biotin-VEKTRCQHERE; SEQ ID NO: 10) dissolved in assay buffer (50 mM Tris-BTB, 8 g/L NaCl, pH 8.0). The plate was incubated for 30 min at 20°C in darkness and was subsequently washed five times in washing buffer (20 mM Tris, 50 mM NaCl, pH 7.2). 20 µl of selection-peptide (VEKTRCQHERE; SEQ ID NO: 11) or sample (e.g. serum) was added to appropriate wells. This was followed by immediately adding 100 µl of monoclonal antibody dissolved in assay-buffer to a concentration of 20 ng/ml. The plate was incubated for 3h at 20°C followed by x5 wash in washing buffer. Then, 100 µl of goat anti-mouse POD-conjugated IgG antibody (Thermo Scientific, Waltham, MA, USA, cat. #31437) diluted 1:6000 in assay buffer (final concentration of 130 ng/ml) was added to each well. The plate was incubated for 1h at 20°C followed by five washes in washing buffer. Next, 100 µL tetra-methyl-benzinidine (Kem-En-Tec, Taastrup, Denmark, cat. #438OH) was added, the plate incubated for 15 min at 20°C, then 100 µl of stopping solution (1% H₂SO₄) was added. All incubation steps were performed while the plate was shaking with 300 rpm. Finally, the optical density was measured in a VersaMax ELISA microplate reader at 450 nm with 650 nm as reference. A 4-parametric mathematical fit model was used to plot a calibration curve. Data were analyzed using the SoftMax Pro v.6.3 software.

### Technical evaluation of the NIC ELISA

The lower limit of detection (LLOD) was determined from 21 measurements of the zero sample (assay buffer) and was calculated as the mean + three standard deviations (SD). The upper limit of detection (ULOD) was determined from ten independent runs of the highest selection peptide concentration employed in the standard curve and calculated as the mean back-calibration concentration + three SD. The lower limit of quantification (LLOQ) was calculated as the highest NIC2-levels quantifiable in serum with a coefficient of variation below 30% reproduced from three independent runs of serum samples diluted stepwise. The intra-assay variation was calculated as the mean coefficient of variance (CV %) within plates, and the inter-assay variation was calculated as the mean CV % between plates. The inter- and intra-assay variations were determined by ten independent runs of eight quality control samples and two internal controls covering the detection range (LLOD-ULOD), with each run consisting of double-determinations of the samples. The eight samples included two human serum samples, two animal serum samples spiked with the selection peptide and four pools of human serum samples spiked with the selection peptide. Two-fold dilutions of human serum and plasma-EDTA samples were used to calculate linearity as a percentage of dilution-recovery of the undiluted sample. Accuracy was determined by spiking human serum and plasma-EDTA samples with two-fold dilutions of the selection peptide, and calculating the percentage spiking-recovery using the expected concentration (serum and peptide combined) as reference. The effect of repeated freezing and thawing of the samples was determined for three human serum and plasma-EDTA samples in four freeze/thaw cycles. The freeze-thaw recovery was calculated with the first cycle as reference. Analyte stability was determined for three human serum and plasma-EDTA samples after 24h of storage at either 4°C or 20°C. Recovery was calculated with samples stored at -20°C as reference. Interference was determined by adding a low/high content of hemoglobin (0.155/0.310 mM), lipemia/lipids (4.83/10.98 mM) and biotin (30/90 ng/ml) to a serum sample of known concentration. Recovery percentage was calculated with the normal serum sample as reference.

### Patient samples

Serum samples consisted of two cohorts. An overview is given in Table 2.

| **Table 2. Patient characteristics** | | | | | | | |
|---|---|---|---|---|---|---|---|
| **study cohort #1** | **Size** | **Age** | **Gender** | **Tumor stage** | | | |
| | **n** | **median, range** | **Females** | **I** | **II** | **III** | **IV** |
| **Controls** | 8 | 55, 44-65 | 6 | - | - | - | - |
| **Breast cancer** | 8 | 55, 34-62 | 8 | - | 3 | 5 | - |
| **NSCLC** | 8 | 62, 47-77 | 1 | 1 | 2 | 3 | 2 |
| **#2** | **n** | **median, range** | **Females** | **I** | **II** | **III** | **IV** |
| **Controls** | 43 | 72, 60-82 | 43 | - | - | - | - |
| **Breast cancer** | 13 | 56, 43-69 | 12 | - | 11 | 2 | - |
| **NSCLC** | 12 | 58, 47-80 | 3 | 5 | 3 | 4 | - |
| **SCLC** | 8 | 57, 46-82 | 2 | 2 | 1 | 4 | 1 |

Appropriate Institutional Review Board/Independent Ethical Committee approved sample collection and all the patients filed informed consent. The first cohort was acquired from the commercial vendor Proteogenex (Culver City, CA, USA). The second cohort was a combination of cancer patient samples acquired from the commercial vendor Asterand (Detroit, MI, USA) and healthy controls samples obtained from another study population (reg.no. KA99070gm). According to Danish law, it is not required to get additional ethical approval when measuring biomarkers in previously collected samples. All investigations were carried out in accordance with the Helsinki Declaration.

### Statistical analysis

Serum levels of NIC were compared by the by one-way ANOVA adjusted for multiple comparisons with Holm-Sidak's test. The area under the receiver operating characteristics (AUROC) curves was used to assess the diagnostics power of NIC. Data were considered statistically significant when p<0.05. GraphPad Prism v6.05 and MedCalc Statistical Software v14.8.1 were used to perform statistical analysis.

### RESULTS

### Specificity of the NIC assay

The specificity of the competitive NIC ELISA was evaluated by assessing the percentage of inhibition induced by the selection peptide (VEKTRCQHERE; SEQ ID NO: 11) as compared to an elongated selection peptide (VEKTRCQHEREH; SEQ ID NO: 16), a truncated selection peptide (EVEKTRCQHER; SEQ ID NO: 15), a non-sense selection peptide (APVGGIIGWM; SEQ ID NO: 12) and a non-sense screening peptide (Biotin-APVGGIIGWM; SEQ ID NO: 13) (the latter two corresponds to the cleavage site located within the central part of the G2 domain on nidogen-1). Results are shown in Figure 1A. The selection peptide clearly inhibited the signal in a dose-dependent manner whereas the signal was only slightly inhibited by the truncated, elongated and non-sense peptides at the highest concentrations. No reactivity was detected towards the non-sense screening peptide. In addition, the levels of NIC generated by incubating nidogen-1 with CatS and various MMPs were investigated. As shown in Figure 1B, CatS generated NIC in a time-dependent manner whereas no NIC was generated neither without proteases, nor when incubating with MMP-1, MMP-7 or MMP-9. Approximately 5-fold higher levels of NIC were detected after incubating nidogen-1 with CatS for 24h. Altogether, this indicates that the antibody is specific and that the assay accurately measures nidogen-1 cleaved by CatS at amino acid position 693.

### Technical evaluation of the NIC assay

The overall technical performance of the NIC assay is listed in Table 3.

### Table 3. Technical validation of the NIC-2 assay

| **Technical validation step** | **Results** |
|---|---|
| Detection range (LLOD-ULOD) | 3.1-260 nM |
| Lower limit of quantification (LLOQ) | 3.2 nM |
| Intra-assay variation | 9% |
| Inter-assay variation | 14% |
| Dilution recovery in serum¹ | 104% (80-115%) |
| Dilution recovery in plasma-EDTA¹ | 92% (81-100%) |
| Spiking recovery in serum¹ | 91% (83-105%) |
| Spiking recovery in plasma-EDTA¹ | 94% (80-102%) |
| Freeze-thaw recovery in serum¹ | 95% (89-101%) |
| Freeze-thaw recovery in plasma-EDTA¹ | 85% (78-101%) |
| Analyte stability serum 24h, 4°C / 20°C¹ | 88% (80-94%) / 82% (71-87%) |
| Analyte stability plasma-EDTA 24h, 4°C / 20°C¹ | 90% (86-94%) / 98% (95-105%) |
| Interference Biotin, low / high¹ | 98% / 118% |
| Interference Lipemia, low / high¹ | 93% / 81% |
| Interference Hemoglobin, low / high¹ | 105% / 101% |
| ¹Percentages are reported as mean with range shown in brackets | |

The assay had a LLOD of 3.1 nM and an ULOD of 260 nM. The LLOQ was 3.2 nM. Intra-assay variation was 9% and the inter-assay variation was 14%; below the acceptance level of 10% and 15%, respectively. All analyte recoveries were accepted if within 100±20%. The dilution recovery was 104% and 92% for serum and plasma-EDTA, respectively, indicating good linearity when diluting the samples. Spiking recovery for the standard peptide was also acceptable in serum (91%) and plasma-EDTA (94%) indicating that these sample matrices do not affect assay response. The analyte was recovered in both serum and plasma-EDTA after four freeze-thaw cycles with 95% and 85% recoveries, respectively. The analyte was also recovered after storage at 4°C and 20°C for 24h resulting in 88% and 82% recoveries for serum and 90% and 98% recoveries for plasma-EDTA at 4°C and 20°C, respectively. Together this indicates that the analyte is relatively stable. No interference was detected from either low or high contents of biotin, lipids (lipemia) or hemoglobin, with recoveries ranging from 81%-105%.

### Evaluation of NIC levels in serum from patients with lung cancer, breast cancer, and healthy controls

NIC levels were measured in serum from two different patient cohorts. As shown in Figure 2A, NIC was significantly elevated (p>0.05) in serum from NSCLC patients as compared to healthy controls and breast cancer patients. Median NIC in the NSCLC patients was 20 nM ranging from 13.1-51.1 nM. Median NIC in the breast cancer patients was 13.3 nM ranging from 6.9-17.3 nM and median NIC in the healthy controls was 12.4 nM ranging from 8.9-14.4 nM. As shown in Figure 2B, NIC was significantly elevated in serum from NSCLC patients as compared to healthy controls (p<0.001), breast cancer patients (p<0.01) and patients with SCLC (p<0.05). In this cohort, median NIC in the NSCLC patients was 13.4 nM ranging from 7.6-22.1 nM. Median NIC in the SCLC patients was 10.4 nM ranging from 7.6-13.7 nM, median NIC in the breast cancer patients was 9.8 nM ranging from 3.3-16.2 nM and median NIC in the healthy controls was 9.7 nM ranging from 4.0-15.1 nM.

NIC levels were also assessed according to tumor stage, an important clinical parameter in cancer. Results are shown in Figure 3 for all cancers combined. In detail, all stages of disease had elevated levels of NIC as compared to the healthy controls, whereas no difference could be detected between stages. This indicates that NIC is generated independent of tumor stage.

To analyze the diagnostic power of NIC with respect to NSCLC, both cohorts were pooled and grouped in 'NSCLC' vs. 'all others' and the AUROC calculated. The AUROC was 0.83 (95% confidence intervals: 0.71-0.95), p<0.0001. The ROC curve, as well as the sensitivity and specificity at estimated optimal cut-off value, is shown in Figure 4. These findings indicate that NIC are able to separate NSCLC patients from the other subjects combined. Altogether, the present findings suggest that NIC may be highly associated with NSCLC.

### DISCUSSION

Described herein is the development and validation of a robust competitive NIC ELISA that enables non-invasive measurements of fragments of nidogen-1 degraded specifically by CatS (NIC). Moreover, this is the first time it has been shows that nidogen-1 degraded specifically by CatS has biomarker potential for cancer, in particular NSCLC.

CatS cleavage of nidogen-1 resulted in the release of a specific fragment containing a neo-epitope with a C-terminal sequence VEKTRCQHERE (SEQ ID NO: 7) (NIC).

Elevated NIC levels as measured by the NIC Assay described herein was detected in serum from patients with NSCLC as compared to patients with breast cancer, SCLC and healthy controls. This suggests that NSCLC patients have increased CatS mediated degradation of nidogen-1, which was further backed by the diagnostic accuracy of NIC (AUROC 0.83) for NSCLC when compared to all other subjects. Furthermore, NIC levels may be indicative of a specific pathological event in a patient's tumor, and this pathological event seems to be more associated with NSCLC as compared to the other cancer types analyzed.

As NIC levels were elevated in all stages of disease, especially in NSCLC, this indicates that CatS degradation of nidogen-1 is ongoing on both early and late stages of disease independent of tumor stage and that NIC may be applied in the early onset of disease.

Concerning the efficacy of anti-cancer drugs, ECM modifying drugs are currently being investigated as possible interventions for modulating the tumor microenvironment and obtain tumor shrinkage or more efficient delivery of chemotherapeutic drugs and targeted therapies. Interestingly, it has been shown that antibody-mediated blockage of CatS enhances the efficacy of chemotherapy (19). Moreover, this antibody has been suggested as a direct strategy for the treatment of solid tumors (20). Thus, the NIC assay could be used for predicting which patients are most likely to benefit from such treatment, or for monitoring early efficacy of treatment.

An important parameter of the NIC assay is related to robustness, i.e. technical and analytical evaluation. Technical evaluation of NIC included the establishment of detection range, sensitivity, inter- and intra-assay variation, linearity/precision (dilution-recovery), analyte stability, and interference, which all were within acceptable limits. Analytical evaluation of the NIC assay refers to the specificity of the assay and to whether the assay detects the analyte it was designed to detect in serum/plasma-EDTA. The specificity was evaluated by displacement analysis (Figure 1A) and by testing reactivity towards intact and cleaved nidogen-1 (Figure 1B). Accuracy was evaluated by spiking the analyte into the relevant matrices (serum and plasma-EDTA) and calculating the percentage recovery (also within acceptable limits). Thus, the NIC assay was technically and analytically robust.

In conclusion, CatS degraded nidogen-1 can be quantified in serum by the technically robust NIC assay. NIC levels were elevated in NSCLC patients as compared to breast cancer patients, SCLC patients and healthy controls. The NIC assay may therefore serve as a non-invasive biomarker for cancer and provide important insight into tumor biology.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'. No acknowledgement of any prior published document herein should be taken to be an admission or representation that the teaching thereof was common general knowledge at the date hereof.

### References

1. Lu P, Weaver VM, Werb Z. The extracellular matrix: a dynamic niche in cancer progression. JCell Biol. Breakthrough Breast Cancer Research Unit, University of Manchester, Manchester M20 4BX, England, UK; 2012;196:395-406.
2. LeBleu VS, Macdonald B, Kalluri R. Structure and function of basement membranes. Exp Biol Med. 2007;232:1121-9.
3. Glentis A, Gurchenkov V, Vignjevic DM. Assembly, heterogeneity, and breaching of the basement membranes. Cell Adhes Migr. 2014;8:236-45.
4. Yurchenco PD, Patton BL. Developmental and Pathogenic Mechanisms of Basement Membrane Assembly. Curr Pharm Des. 2009;15:1277-94.
5. Ho MSP, Böse K, Mokkapati S, Nischt R, Smyth N. Nidogens - Extracellular matrix linker molecules. Microsc Res Tech. 2008;71:387-95.
6. Chung AE, Durkin ME. Entactin: structure and function. Am J Respir Cell Mol Biol. 1990;3:275-82.
7. Bader BL, Smyth N, Nedbal S, Miosge N, Baranowsky A, Mokkapati S, et al. Compound Genetic Ablation of Nidogen 1 and 2 Causes Basement Membrane Defects and Perinatal Lethality in Mice. Mol Cell Biol. 2005;25:6846-56.
8. Pujuguet P, Simian M, Liaw J, Timpl R, Werb Z, Bissell MJ. Nidogen-1 regulates laminin-1-dependent mammary-specific gene expression. J Cell Sci. 2000;113:849-58.
9. Sage J, Leblanc-Noblesse E, Nizard C, Sasaki T, Schnebert S, Perrier E, et al. Cleavage of Nidogen-1 by Cathepsin S Impairs Its Binding to Basement Membrane Partners. PLoS One. 2012;7:e43494.
10. Mohamed MM, Sloane BF. Cysteine cathepsins: multifunctional enzymes in cancer. Nat Rev Cancer. 2006;6:764-75.
11. Sevenich L, Bowman RL, Mason SD, Quail DF, Elie BT, Brogi E, et al. Analysis of tumor- and stroma-supplied proteolytic networks reveals a brain metastasis-promoting role for cathepsin S. NatCell Biol. 2014;16:876-88.
12. Fan Q, Wang X, Zhang H, Li C, Fan J, Xu J. Silencing cathepsin S gene expression inhibits growth, invasion and angiogenesis of human hepatocellular carcinoma in vitro. Biochem Biophys Res Commun. Elsevier Inc.; 2012;425:703-10.
13. Yixuan Y, Kiat LS, Yee CL, Huiyin L, Yunhao C, Kuan CP, et al. Cathepsin S mediates gastric cancer cell migration and invasion via a putative network of metastasis-associated proteins. J Proteome Res. 2010;9:4767-78.
14. Li L, Zhang Y, Li N, Feng L, Yao H, Zhang R, et al. Nidogen-1: a candidate biomarker for ovarian serous cancer. Jpn J Clin Oncol. 2015;45:176-82.
15. Combet C, Blanchet C, Geourjon C, Deléage G. NPS@: network protein sequence analysis. Trends Biochem Sci. 2000;25:147-50.
16. Gefter ML, Margulies DH, Scharff MD. A simple method for polyethylene glycol-promoted hybridization of mouse myeloma cells. Somat Genet. 1977;3:231-6.
17. Mayer U, Mann K, Timpl R, Murphy G. Sites of nidogen cleavage by proteases involved in tissue homeostasis and remodelling. Eur. J. Biochem. 1993. page 877-84.
18. Sires UI, Griffin GL, Broekelmann TJ, Mecham RP, Murphy G, Chung a E, et al. Degradation of entactin by matrix metalloproteinases. J Biol Chem. 1993;268:2069-74.
19. Burden RE, Gormley JA, Kuehn D, Ward C, Kwok HF, Gazdoiu M, et al. Inhibition of Cathepsin S by Fsn0503 enhances the efficacy of chemotherapy in colorectal carcinomas. Biochimie. Elsevier Masson SAS; 2012;94:487-93.
20. Vázquez R, Astorgues-Xerri L, Bekradda M, Gormley J, Buick R, Kerr P, et al. Fsn0503h antibody-mediated blockade of cathepsin S as a potential therapeutic strategy for the treatment of solid tumors. Biochimie. 2014;108:101-7.
21. Kos J, Sekirnik A, Kopitar G, Cimerman N, Kayser K, Stremmer A, Fiehn W, Werle B, Cathepsin S in tumours, regional lymph nodes and sera of patients with lung cancer: relation to prognosis. British J. Cancer. 2001; 85:1193-1200.
22. Kalluri R. Basement membranes: structure, assembly and role in tumour angiogenesis. Nat Rev Cancer. 2003; 3(6):422-33.
23. Tan GJ, Peng ZK, Lu JP, Tang FQ. Cathepsins mediate tumor metastasis. World J Biol Chem. 2013; 4(4):91-101.
24. LIN LI ET AL: JAPANESE JOURNAL OF CLINICAL ONCOLOGY., vol. 45, no. 2, 6 November 2014 (2014-11-06), pages 176-182,JP ISSN: 0368-2811

## Claims

1. A method of diagnosis or of quantitation of cancer comprising conducting an immunoassay on a biofluid sample obtained from a patient to measure fragments of Nidogen-1 having an N- or C-terminal neo-epitope formed by cleavage of Nidogen-1 by Cathepsin-S, said fragments being naturally present in said sample, and associating an elevation of said measure in said patient above a normal level with the presence or extent of cancer, wherein said immunoassay is conducted by a method comprising:
contacting the fragments of Nidogen-1 having said N- or C-terminal neo epitope that are naturally present in said sample with an immunological binding partner specifically reactive with the N- or C-terminal neo-epitope but not reactive with intact Nidogen-1, and measuring the extent of binding of the N- or C-terminal neo-epitope to said immunological binding partner to measure therein fragments comprising said neo-epitope.

2. A method according to claim 1, wherein the cancer is breast cancer or non-small cell lung cancer.

3. A method according to claims 1 to 2, wherein the immunological binding partner is specifically reactive with a C-terminal neo-epitope selected from the group consisting of:
...CQHERE (SEQ ID NO: 1)
...YSLLPL (SEQ ID NO: 2)
...IIRQDL (SEQ ID NO: 3)
or is specifically reactive with an N-terminal neo-epitope selected from the group consisting of:
APVGGI... (SEQ ID NO: 4)
HILGAA... (SEQ ID NO: 5)
GSPEGI... (SEQ ID NO: 6)

4. A method according to claims 1 to 3, wherein the immunological binding partner is specifically reactive with the C-terminal neo-epitope VEKTRCQHERE-COOH (SEQ ID NO: 7).

5. A method according to claim 4, wherein the immunological binding partner does not react with a truncated C-terminal sequence VEKTRCQHER-COOH (SEQ ID NO: 8) and/or wherein the immunological binding partner does not react with an elongated C-terminal sequence VEKTRCQHEREH-COOH (SEQ ID NO: 9)

6. A method according to claims 1 to 5, wherein the immunological binding partner is a polyclonal antibody or a monoclonal antibody.

7. A method for evaluating the efficacy of an anti-cancer drug, wherein said method comprises
conducting an immunoassay to quantify the amount of fragments of Nidogen-1 having an N- or C-terminal neo-epitope formed by cleavage of Nidogen-1 by Cathepsin-S in at least two biological samples, said biological samples having been obtained from a subject at a first time point and at at least one subsequent time point during a period of administration of the anti-cancer drug to said subject, and wherein a reduction in the quantity of said fragments of Nidogen-1 having an N- or C-terminal neo-epitope formed by cleavage of Nidogen-1 by Cathepsin-S from said first time point to said at least one subsequent time point during the period of administration of the anti-cancer drug is indicative of an efficacious anti-cancer drug,
wherein said immunoassay is conducted by a method comprising contacting the fragments of Nidogen-1 having said N- or C-terminal neo epitope that are naturally present in said samples with an immunological binding partner specifically reactive with the N- or C-terminal neo-epitope but not reactive with intact Nidogen-1, and measuring the extent of binding of the N- or C-terminal neo-epitope to said immunological binding partner to measure therein fragments comprising said neo-epitope.

8. A method according to claim 7, wherein the immunological binding partner is specifically reactive with a C-terminal neo-epitope selected from the group consisting of:
...CQHERE (SEQ ID NO: 1)
...YSLLPL (SEQ ID NO: 2)
...IIRQDL (SEQ ID NO: 3)
or is specifically reactive with an N-terminal neo-epitope selected from the group consisting of:
APVGGI... (SEQ ID NO: 4)
HILGAA... (SEQ ID NO: 5)
GSPEGI... (SEQ ID NO: 6)

9. A method according to claims 7 or 8, wherein the immunological binding partner is specifically reactive with the C-terminal neo-epitope VEKTRCQHERE-COOH (SEQ ID NO: 7).

10. A method according to claim 9, wherein the immunological binding partner does not react with a truncated C-terminal sequence VEKTRCQHER-COOH (SEQ ID NO: 8) and/or wherein the immunological binding partner does not react with an elongated C-terminal sequence VEKTRCQHEREH-COOH (SEQ ID NO: 9).

11. A method according to claims 7 to 10, wherein the immunological binding partner is a polyclonal antibody or a monoclonal antibody.

12. A diagnostic kit for use in a method according to claims 1 to 11, the kit comprising an immunological binding partner specifically reactive with an N- or C-terminal neo-epitope formed by cleavage of Nidogen-1 by Cathepsin-S but not reactive with intact Nidogen-1, and at least one of the following:
- a streptavidin coated 96 well plate
- a biotinylated peptide corresponding to the amino acid sequence of the N- or C-terminal neo-epitope, with an optional linker located between the biotin residue and the peptide
- a biotinylated secondary antibody for use in a sandwich immunoassay
- a calibrator peptide corresponding to the amino acid sequence of the N- or C-terminal neo-epitope
- an antibody HRP labeling kit
- an antibody radiolabeling kit
- an assay visualization kit

13. The diagnostic kit according to claim 12, wherein the diagnostic kit comprises a biotinylated peptide Biotin-L-VEKTRCQHERE-COOH (SEQ ID NO: 10), wherein L is an optional linker, and a calibrator peptide comprising the C-terminal sequence VEKTRCQHERE-COOH (SEQ ID NO: 11).

## Patentansprüche

1. Verfahren zur Diagnose oder Quantifizierung von Krebs, umfassend Durchführen eines Immunoassays an einer von einem Patienten erlangten Probe biologischer Flüssigkeit, um Fragmente von Nidogen-1 mit einem N- oder C-terminalen Neo-Epitop zu messen, das durch Spaltung von Nidogen-1 durch Cathepsin-S gebildet wird, wobei die Fragmente natürlicherweise in der Probe vorhanden sind, und Verknüpfen einer Erhöhung der Messung bei dem Patienten über einen normalen Wert hinaus mit dem Vorhandensein oder Ausmaß von Krebs, wobei der Immunoassay durch ein Verfahren durchgeführt wird, das Folgendes umfasst:
Inkontaktbringen der Fragmente von Nidogen-1 mit dem N- oder C-terminalen Neo-Epitop, die natürlicherweise in der Probe vorhanden sind, mit einem immunologischen Bindungspartner, der spezifisch mit dem N- oder C-terminalen Neo-Epitop, aber nicht mit intaktem Nidogen-1 reaktiv ist, und Messen des Ausmaßes der Bindung des N- oder C-terminalen Neo-Epitops an den immunologischen Bindungspartner, um darin Fragmente zu messen, die das Neo-Epitop umfassen.

2. Verfahren nach Anspruch 1, wobei der Krebs Brustkrebs oder nicht-kleinzelliger Lungenkrebs ist.

3. Verfahren nach Anspruch 1 bis 2, wobei der immunologische Bindungspartner spezifisch mit einem C-terminalen Neo-Epitop reaktiv ist, das ausgewählt ist aus der Gruppe bestehend aus:
...CQHERE (SEQ ID NO: 1)
...YSLLPL (SEQ ID NO: 2)
...IIRQDL (SEQ ID NO: 3)
oder spezifisch mit einem N-terminalen Neo-Epitop reaktiv ist, das ausgewählt ist aus der Gruppe bestehend aus:
APVGGI ... (SEQ ID NO: 4)
HILGAA... (SEQ ID NO: 5)
GSPEGI... (SEQ ID NO: 6)

4. Verfahren nach einem der Ansprüche 1 bis 3, wobei der immunologische Bindungspartner spezifisch mit dem C-terminalen Neo-Epitop VEKTRCQHERE-COOH (SEQ ID NO: 7) reaktiv ist.

5. Verfahren nach Anspruch 4, wobei der immunologische Bindungspartner nicht mit einer verkürzten C-terminalen Sequenz VEKTRCQHER-COOH (SEQ ID NO: 8) reagiert und/oder wobei der immunologische Bindungspartner nicht mit einer verlängerten C-terminalen Sequenz VEKTRCQHEREH-COOH (SEQ ID NO: 9) reagiert.

6. Verfahren nach einem der Ansprüche 1 bis 5, wobei der immunologische Bindungspartner ein polyklonaler Antikörper oder ein monoklonaler Antikörper ist.

7. Verfahren zur Bewertung der Wirksamkeit eines Krebsmedikaments, wobei das Verfahren Folgendes umfasst:
Durchführen eines Immunoassays, um die Menge an Fragmenten von Nidogen-1 mit einem N- oder C-terminalen Neo-Epitop, das durch die Spaltung von Nidogen-1 durch Cathepsin-s gebildet wird, in mindestens zwei biologischen Proben zu quantifizieren, wobei die biologischen Proben von einem Probanden zu einem ersten Zeitpunkt und zu mindestens einem nachfolgenden Zeitpunkt während eines Zeitraums der Verabreichung des Krebsmedikaments an den Probanden erlangt wurden, und wobei eine Verringerung der Menge der Fragmente von Nidogen-1 mit einem N- oder C-terminalen Neo-Epitop, das durch die Spaltung von Nidogen-1 durch Cathepsin-s gebildet wird, von dem ersten Zeitpunkt bis zu dem mindestens einen nachfolgenden Zeitpunkt während des Verabreichungszeitraums des Krebsmedikaments auf ein wirksames Krebsmedikament hinweist,
wobei der Immunoassay nach einem Verfahren durchgeführt wird, das Inkontaktbringen der Fragmente von Nidogen-1 mit dem N- oder C-terminalen Neoepitop, die natürlicherweise in den Proben vorhanden sind, mit einem immunologischen Bindungspartner, der spezifisch mit dem N- oder C-terminalen Neoepitop, aber nicht mit intaktem Nidogen-1 reaktiv ist, und Messen des Ausmaßes der Bindung des N- oder C-terminalen Neoepitops an den immunologischen Bindungspartner umfasst, um darin Fragmente zu messen, die das Neoepitop umfassen.

8. Verfahren nach Anspruch 7, wobei der immunologische Bindungspartner spezifisch mit einem C-terminalen Neo-Epitop reagiert, das ausgewählt ist aus der Gruppe bestehend aus:
...CQHERE (SEQ ID NO: 1)
...YSLLPL (SEQ ID NO: 2)
...IIRQDL (SEQ ID NO: 3)
oder spezifisch mit einem N-terminalen Neo-Epitop reaktiv ist, das ausgewählt ist aus der Gruppe bestehend aus:
APVGGI... (SEQ ID NO: 4)
HILGAA... (SEQ ID NO: 5)
GSPEGI... (SEO ID NO: 6)

9. Verfahren nach Anspruch 7 oder 8, wobei der immunologische Bindungspartner spezifisch mit dem C-terminalen Neo-Epitop VEKTRCQHERE-COOH (SEQ ID NO: 7) reaktiv ist.

10. Verfahren nach Anspruch 9, wobei der immunologische Bindungspartner nicht mit einer verkürzten C-terminalen Sequenz VEKTRCQHER-COOH (SEQ ID NO: 8) reagiert und/oder wobei der immunologische Bindungspartner nicht mit einer verlängerten C-terminalen Sequenz VEKTRCQHEREH-COOH (SEQ ID NO: 9) reagiert.

11. Verfahren nach Anspruch 7 bis 10, wobei der immunologische Bindungspartner ein polyklonaler Antikörper oder ein monoklonaler Antikörper ist.

12. Diagnose-Set zur Verwendung in einem Verfahren nach Anspruch 1 bis 11, wobei das Set einen immunologischen Bindungspartner, der spezifisch mit einem N- oder C-terminalen Neo-Epitop, das durch Spaltung von Nidogen-1 durch Cathepsin-s gebildet wird, reaktiv ist, aber nicht mit intaktem Nidogen-1 reaktiv ist, und mindestens eines von Folgendem umfasst:
- eine mit Streptavidin beschichtete 96-Well-Platte
- ein biotinyliertes Peptid, das der Aminosäuresequenz des N- oder C-terminalen Neo-Epitops entspricht, mit einem optionalen Linker zwischen dem Biotin-Rest und dem Peptid
- einen biotinylierten Sekundärantikörper zur Verwendung in einem Sandwich-Immunoassay
- ein Kalibrierungspeptid, das der Aminosäuresequenz des N- oder C-terminalen Neo-Epitops entspricht
- ein Antikörper-HRP-Markierungsset
- ein Antikörper-Radiomarkierungsset
- ein Assay-Visualisierungsset

13. Diagnoseset nach Anspruch 12, wobei das Diagnoseset ein biotinyliertes Peptid Biotin-L-VEKTRCQHERE-COOH (SEQ ID NO: 10), wobei L ein optionaler Linker ist, und ein Kalibratorpeptid mit der C-terminalen Sequenz VEKTRCQHERE-COOH (SEQ ID NO: 11) umfasst.

## Revendications

1. Procédé de diagnostic ou de quantification de cancer, comprenant l'exécution d'un dosage immunologique dans un échantillon de liquide biologique obtenu auprès d'un patient pour doser des fragments de nidogène-1 présentant un néo-épitope N- ou C-terminal formé par clivage du nidogène-1 par la cathepsine S, lesdits fragments étant présents naturellement dans ledit échantillon, et l'association d'une élévation dudit dosage au-dessus d'un taux normal avec la présence ou l'étendue de cancer chez ledit patient, dans lequel ledit dosage immunologique est exécuté par un procédé comprenant les étapes consistant à :
mettre les fragments de nidogène-1 présentant ledit néo-épitope N- ou C-terminal, qui sont présents naturellement dans ledit échantillon, en contact avec un partenaire de liaison immunologique spécifiquement réactif avec le néo-épitope N- ou C-terminal mais non réactif avec le nidogène-1 intact, et mesurer le degré de liaison du néo-épitope N- ou C-terminal audit partenaire de liaison immunologique afin de doser dans cet échantillon les fragments comprenant ledit néo-épitope.

2. Procédé selon la revendication 1, dans lequel le cancer est le cancer du sein ou le cancer du poumon non à petites cellules.

3. Procédé selon les revendications 1 et 2, dans lequel le partenaire de liaison immunologique est spécifiquement réactif avec un néo-épitope C-terminal choisi dans le groupe constitué par :
...CQHERE (SEQ ID NO: 1)
...YSLLPL (SEQ ID NO: 2)
...IIRQDL (SEQ ID NO: 3)
ou est spécifiquement réactif avec un néo-épitope N-terminal choisi dans le groupe constitué par :
APVGGI... (SEQ ID NO: 4)
HILGAA... (SEQ ID NO: 5)
GSPEGI... (SEQ ID NO: 6).

4. Procédé selon les revendications 1 à 3, dans lequel le partenaire de liaison immunologique est spécifiquement réactif avec le néo-épitope C-terminal VEKTRCQHERE-COOH (SEQ ID NO: 7).

5. Procédé selon la revendication 4, dans lequel le partenaire de liaison immunologique ne réagit pas avec une séquence C-terminale tronquée VEKTRCQHER-COOH (SEQ ID NO: 8) et/ou dans lequel le partenaire de liaison immunologique ne réagit pas avec une séquence C-terminale prolongée VEKTRCQHEREH-COOH (SEQ ID NO: 9).

6. Procédé selon les revendications 1 à 5, dans lequel le partenaire de liaison immunologique est un anticorps polyclonal ou un anticorps monoclonal.

7. Procédé d'évaluation de l'efficacité d'un médicament anticancéreux, ledit procédé comprenant
exécuter un dosage immunologique pour déterminer la quantité de fragments de nidogène-1 présentant un néo-épitope N- ou C-terminal formé par clivage du nidogène-1 par la cathepsine S dans au moins deux échantillons biologiques, lesdits échantillons biologiques ayant été obtenus auprès d'un sujet à un premier instant et à au moins un instant subséquent pendant une période d'administration du médicament anticancéreux audit sujet, et une diminution de la quantité desdits fragments de nidogène-1 présentant un néo-épitope N- ou C-terminal formé par clivage du nidogène-1 par la cathepsine S depuis ledit premier instant jusqu'audit au moins un instant subséquent pendant la période d'administration du médicament anticancéreux étant indicative d'un médicament anticancéreux efficace,
ledit dosage immunologique étant effectué par un procédé comprenant les étapes consistant à mettre les fragments de nidogène-1 présentant ledit néo-épitope N- ou C-terminal, qui sont présents naturellement dans lesdits échantillons, en contact avec un partenaire de liaison immunologique spécifiquement réactif avec le néo-épitope N- ou C-terminal mais non réactif avec le nidogène-1 intact, et mesurer le degré de liaison du néo-épitope N- ou C-terminal audit partenaire de liaison immunologique afin de doser dans ces échantillons les fragments comprenant ledit néo-épitope.

8. Procédé selon la revendication 7, dans lequel le partenaire de liaison immunologique est spécifiquement réactif avec un néo-épitope C-terminal choisi dans le groupe constitué par :
...CQHERE (SEQ ID NO: 1)
...YSLLPL (SEQ ID NO: 2)
...IIRQDL (SEQ ID NO: 3)
ou est spécifiquement réactif avec un néo-épitope N-terminal choisi dans le groupe constitué par :
APVGGI... (SEQ ID NO: 4)
HILGAA... (SEQ ID NO: 5)
GSPEGI... (SEQ ID NO: 6).

9. Procédé selon la revendication 7 ou 8, dans lequel le partenaire de liaison immunologique est spécifiquement réactif avec le néo-épitope C-terminal VEKTRCQHERE-COOH (SEQ ID NO: 7).

10. Procédé selon la revendication 9, dans lequel le partenaire de liaison immunologique ne réagit pas avec une séquence C-terminale tronquée VEKTRCQHER-COOH (SEQ ID NO: 8) et/ou dans lequel le partenaire de liaison immunologique ne réagit pas avec une séquence C-terminale prolongée VEKTRCQHEREH-COOH (SEQ ID NO: 9).

11. Procédé selon les revendications 7 à 10, dans lequel le partenaire de liaison immunologique est un anticorps polyclonal ou un anticorps monoclonal.

12. Kit de diagnostic pour utilisation dans un procédé selon les revendications 1 à 11, le kit comprenant un partenaire de liaison immunologique spécifiquement réactif avec un néo-épitope N- ou C-terminal formé par clivage du nidogène-1 par la cathepsine S mais non réactif avec le nidogène-1 intact, et au moins un(e) parmi les suivant(e)s :
- une plaque à 96 puits tapissée avec de la streptavidine
- un peptide biotinylé correspondant à la séquence d'acides aminés du néo-épitope N- ou C-terminal, avec un segment de liaison optionnel situé entre le résidu de biotine et le peptide
- un anticorps secondaire biotinylé pour utilisation dans un dosage immunologique en sandwich
- un peptide d'étalonnage correspondant à la séquence d'acides aminés du néo-épitope N- ou C-terminal
- un kit de marquage HRP d'anticorps
- un kit de marquage radioactif d'anticorps
- un kit de visualisation de dosage.

13. Kit de diagnostic selon la revendication 12, le kit de diagnostic comprenant un peptide biotinylé biotine-L-VEKTRCQHERE-COOH (SEQ ID NO: 10), où L est un segment de liaison optionnel, et un peptide d'étalonnage comprenant la séquence C-terminale VEKTRCQHERE-COOH (SEQ ID NO: 11).
